# EUROPÄISCHE PATENTSCHRIFT

(11) **EP 1 854 434 B1**
(45) Veröffentlichungstag und Bekanntmachung des Hinweises auf die Patenterteilung: **11.01.2012**
(21) Anmeldenummer: 07008413.2
(22) Anmeldetag: 25.04.2007
(51) Int. Cl.: A61F 5/01, A61F 5/30

(54) **Gelenkorthese**
Jointed orthesis
Orthèse articulatoire

(30) Priorität: 09.05.2006 DE 102006021789
(43) Veröffentlichungstag der Anmeldung: 14.11.2007
(73) Patentinhaber: Otto Bock HealthCare GmbH, 37115 Duderstadt (DE)
(72) Erfinder: Kahlmeyer, Guido, 37308 Siemerode (DE); Lidolt, Klaus, 37115 Duderstadt (DE); Wagner, Helmut, 37115 Duderstadt (DE); Mühlenberend, Andreas, 04229 Leipzig (DE)
(74) Vertreter: Stornebel, Kai

(56) Entgegenhaltungen:
- DE-A1- 19 650 782
- US-A- 1 918 590
- US-A- 4 997 438
- US-A- 5 772 618
- US-A- 5 807 294

## Beschreibung

Die Erfindung betrifft eine Gelenkorthese mit einem Oberteil und einem Unterteil, die über eine Gelenkeinrichtung um zumindest eine Schwenkachse schwenkbar miteinander verbunden sind, wobei das Oberteil und das Unterteil Einrichtungen zum Festlegen der Gelenkorthese an einem Körperteil, insbesondere an einer Extremität aufweisen, und einer an der Orthese befestigten Pelotte, die in einem Zwischenraum zwischen der Gelenkeinrichtung und dem Körperteil angeordnet ist. Insbesondere ist die Gelenkorthese zur Anwendung als eine Kniegelenkorthese geeignet.

Gelenkorthesen werden zur Unterstützung und Stabilisierung von Gelenken in dem menschlichen Körper eingesetzt. Sie unterstützen dabei die physiologisch korrekte Bewegung des Gelenkes, beispielsweise nach einer Operation oder einer Gelenkschädigung. Darüber hinaus können Gelenkorthesen zur Begrenzung des Beugewinkels eingesetzt werden, um im Rahmen von Rehabilitationsmaßnahmen eine abgestufte also Anpassung an den maximalen Beugewinkel zu ermöglichen.

Gelenkorthesen werden dazu über Riemen an den durch das Gelenk verbundenen Körperteilen oder Extremitäten festgelegt, beispielsweise an dem Oberschenkel und dem Unterschenkel, dem Unterschenkel und dem Fuß oder dem Oberarm und dem Unterarm. Das Oberteil und das Unterteil weisen dabei einen Rahmen auf, der vorzugsweise aus zumindest einer Schiene besteht. Eine gelenkige Verbindung von Oberteil und Unterteil bzw. der Schienen des Oberteils und des Unterteils ermöglicht ein Verschwenken um zumindest eine Schwenkachse. Neben einseitig angeordneten Rahmenteilen können diese auch medial und lateral neben dem zu unterstützenden Gelenk angeordnet sein.

Im Bereich des Gelenkspaltes oder der Gelenkachse des zu unterstützenden Gelenkes sind Pelotten angeordnet, die den Raum zwischen der Gelenkeinrichtung bzw. der Rahmenteile des Oberteils oder des Unterteils überbrücken und eine Kopplung der Gelenkorthese mit dem Körperteil oder den Extremitäten bewirken. Die Befestigung solcher Pelotten erfolgt in der Regel über einen sogenannten Klettverschluss oder über eine Verschraubung. Eine Einstellung und Anpassung an die individuellen Gegebenheiten des zu unterstützenden Gelenkes erfolgt dabei über Madenschrauben. Zur Anpassung an die unterschiedlichen Ausdehnungen der Extremitäten oder im Verlauf der Rehabilitation bei abschwellenden Gelenken ist ein aufwendiger Austausch der Pelotte oder der daran befestigten Polster notwendig. Eine solche Orthese ist aus der US 5,807,294 bekannt.

Aufgabe der vorliegenden Erfindung ist es, eine Gelenkorthese bereitzustellen, mit der eine schnelle und gleichzeitig stabile Anpassung an die individuellen physiologischen Gegebenheiten erreicht werden kann.

Erfindungsgemäß wird diese Aufgabe durch eine Gelenkorthese mit den Merkmalen des Anspruchs 1 gelöst. Vorteilhafte Ausgestaltungen und Weiterbildungen der Erfindung sind in den Unteransprüchen aufgeführt.

Die erfindungsgemäße Gelenkorthese mit einem Oberteil und einem Unterteil um zumindest eine Schwenkachse schwenkbar miteinander verbunden sind, wobei das Oberteil und das Unterteil Einrichtungen zum Festlegen der Gelenkorthese an einem Körperteil, insbesondere an einer Extremität, aufweisen und einer an der Orthese befestigten Pelotte, die in dem Raum zwischen der Gelenkeinrichtung und dem Körperteil angeordnet ist, sieht vor, dass die Gelenkorthese und die Pelotte oder zumindest ein Kopplungselement Formschlusselemente aufweisen, die eine Clipsverbindung zur Befestigung der Pelotte an der Gelenkorthese ausbilden. Die clipsbare Ausgestaltung der Pelotte oder eines Kopplungselementes zwischen der Pelotte und der Orthese oder der Gelenkeinrichtung ermöglicht ein leichtes Auswechseln der Pelotte, um verschiedene Abstände zwischen dem Köperteil und der Gelenkeinrichtung zu überbrücken und dadurch eine leichte Anpassbarkeit an das jeweilige Gelenk zu ermöglichen. Die Pelotte kann unmittelbar oder über ein oder mehrere Kopplungselemente, z.B. einen Pelottenträger, der die Pelotte aufnimmt, und einen Adapterring an der Gelenkorthese oder der Gelenkeinrichtung befestigt werden.

Die Erfindung sieht weiterhin vor, dass die Pelotte oder eine Pelottenhalterung an der Gelenkorthese angeordnet ist, indem an der Gelenkeinrichtung ein Hinterschnitt zur Aufnahme der Pelotte oder des Kopplungselementes angeordnet oder ausgebildet ist. Das Kopplungselement oder die Pelotte sind bevorzugt so ausgebildet, dass die Schwenkachse oder Schwenkachsen oder der Gelenkspalt, der zwischen dem Oberteil und dem Unterteil ausgebildet ist, im Wesentlichen abgedeckt wird. Dazu kann vorgesehen sein, dass der die Pelotte oder das Kopplungselement die Schwenkachse bzw. Schwenkachsen und den Gelenkspalt zwischen dem Oberteil und dem Unterteil umgibt oder einschließt. Dazu ist der Kontaktbereich oder der Haltebereich der Pelotte oder des Kopplungselementes bevorzugt als ein Ring oder ringartig oder als Steg ausgebildet, an dem die Formschlusselemente vorhanden sind. Durch eine entsprechende Querschnittsformung der Pelotte oder des Kopplungselementes, beispielsweise durch einen umlaufenden Hinterschnitt, kann der gesamte Kontakt- oder Haltebereich der Pelotte oder des Kopplungselementes als Formschlusselement ausgebildet sein.

Eine Weiterbildung der Erfindung sieht vor, dass das Oberteil und das Unterteil Verzahnungen aufweisen, die bevorzugt an den Rahmenteilen des Oberteils und des Unterteils ausgebildet sind. Diese Verzahnungen kämmen miteinander, so dass eine abrollende Bewegung des Oberteils auf dem Unterteil realisiert wird. Eine solche Gelenkeinrichtung ist insbesondere bei Kniegelenkorthesen sinnvoll. Dabei ist die Gelenkeinrichtung so ausgebildet, dass an dem Oberteil und dem Unterteil je eine Schwenkachse vorhanden ist. Die Pelotte oder das Kopplungselement deckt dabei sowohl die Schwenkachsen als auch den Verzahnungsbereich ab.

Um zu vermeiden, dass bei notwendigen Anpassungen die gesamte Pelotte ausgetauscht werden müssen, sieht eine Weiterbildung der Erfindung vor, dass das Kopplungselement als ein Pelottenträger oder ein Adapter, insbesondere einen Adapterring ausgebildet ist, der zwischen der Gelenkeinrichtung und der Pelotte einzuclipsen ist, um die laterale Distanz zwischen der Pelotte und der Gelenkeinrichtung überbrücken zu können. Der Adapter bzw. Adapterring und der Pelottenträger weist dabei sowohl mit der Pelotte als auch mit der Gelenkeinrichtung korrespondierende Formschlusselemente auf, so dass durch Austausch des Adapters oder des Pelottenträgers eine einfache Anpassung erfolgen kann. Neben der ringförmigen Ausgestaltung des Adapters können auch andere Formen und Funktionen durch den Adapter realisiert werden, beispielsweise eine schwenkbare Lagerung der Pelotte oder des Pelottenträgers.

Eine Weiterbildung der Erfindung sieht vor, dass die Pelotte oder das Kopplungselement, also der Pelottenträger oder der Adapterring, einen keilförmigen Querschnitt aufweisen, um eine im Vergleich zu der Schwenkachse schräg gestellte Anlagefläche der Pelotte an der Extremität oder dem Körperteil realisieren zu können.

Ein symmetrischer Aufbau bei einer bilateralen Anordnung von Gelenkeinrichtungen neben dem zu unterstützenden Gelenk erleichtert den Austausch von Pelotten und Kopplungselementen, so dass sowohl medial als auch lateral mit denselben Adaptern und Pelotten gearbeitet werden kann. Dies verringert die zu bevorratende Komponentenanzahl

Eine Weiterbildung der Erfindung sieht vor, dass Anschläge, insbesondere Anschlageinsätze in der Gelenkeinrichtung gelagert sind, um den Schwenkwinkel zwischen Oberteil und Unterteil verstellen zu können. Die Anschläge oder Anschlageinsätze sind an der Gelenkeinrichtung festgelegt, insbesondere eingeclipst, um eine leichte Auswechselbarkeit realisieren zu können. Dadurch ist es möglich, einen werkzeuglosen Austausch der Anschläge vorzunehmen und die Gelenkorthese leicht an den Rehabilitationsfortschritt anzupassen.

Um bei einem Kontakt des Oberteils und des Unterteils mit den Anschlägen ein Herausdrücken dieser aus der Gelenkeinrichtung zu vermeiden, sind die Pelotte oder das Kopplungselement, insbesondere der Adapter bzw. Adapterring und deer Pelottenträgr so ausgebildet, dass die Anschläge oder Anschlageinsätze gesichert werden. Dies wird insbesondere dadurch erreicht, dass die Pelotte oder der Adapter bzw. Pelottenträger im eingeclipsten Zustand die Anschläge formschlüssig sichert, insbesondere umgibt. Dadurch ist es möglich, dass sowohl in Flexionsrichtung als auch Extensionsrichtung ein Herausdrücken der Anschläge wirksam vermieden wird. Ebenfalls tritt kein unbeabsichtigtes Herausfallen der Anschläge auf, was unbeabsichtigte Veränderungen der maximalen Flexions- oder Extensionswinkel vermeidet.

Eine Weiterbildung der Erfindung sieht vor, dass an dem Oberteil oder dem Unterteil Hinterschnitte ausgebildet oder angeordnet sind, die die Anschlagansätze bei Kontakt mit dem Oberteil und dem Unterteil bzw. den Rahmenteilen blockieren. Dies erfolgt durch eine formschlüssige Koppelung der Anschläge mit dem Oberteil oder dem Unterteil und ein Eingreifen der Hinterschnitte in die Anschläge. Die Anschläge können ebenfalls Hinterschnitte aufweisen, die korrespondierend zu den Hinterschnitten an dem Oberteil oder dem Unterteil ausgebildet sind. Bevorzugt sind diese Hinterschnitte zangenartig ausgebildet.

Nachfolgend werden Ausführungsbeispiele der Erfindung anhand der beigefügten Figuren näher erläutert. Es zeigen:
- Figur 1 -: ein Rahmenteil einer Gelenkorthese;
- Figur 2 -: eine Explosionsdarstellung des Rahmenteils gemäß Figur 1;
- Figuren 3a bis 3e -: Darstellungen eines Pelottenträgers;
- Figuren 4a bis 4e -: Darstellungen eines Adapters;
- Figuren 5a und 5b -: Ansichten eines Anschlageinsatzes; sowie
- Figuren 6a und 6b -: Darstellungen einer Variante eines Pelottenträgers.

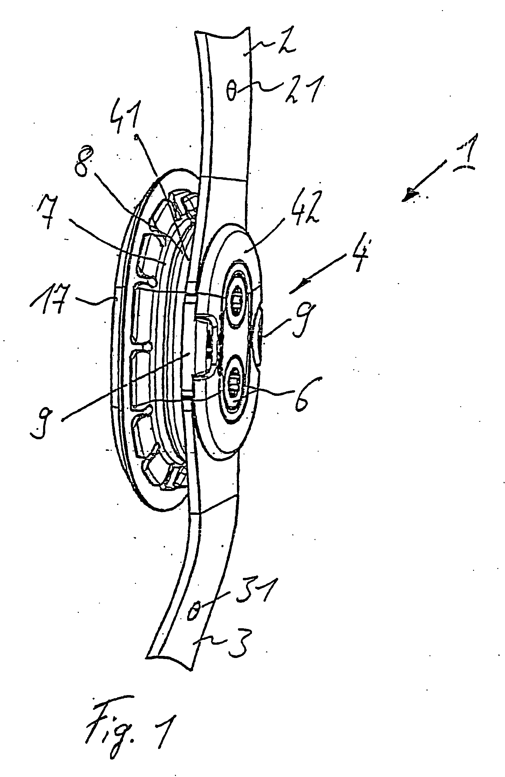

In der Figur 1 ist ein Teil einer Gelenkorthese ohne Befestigungseinrichtungen zur Festlegung an einem Körperteil oder an einer Extremität gezeigt. Dieses Rahmenteil 1 ist aus einem Oberteil 2 und einem Unterteil 3 aufgebaut, die als gebogene Schienen ausgebildet sind. Bevorzugt sind diese Schienen 2, 3 aus einem Leichtmetall hergestellt. An den Bohrungen 21, 31 können Befestigungseinrichtungen, wie Klettverschlüsse oder Riemen sowie Polstereinrichtungen festgelegt werden. Ebenfalls können weitere metallische oder aus Kunststoff bestehende Orthesenkomponenten an den Schienen 2, 3 befestigt werden. Die Schienen 2, 3 sind über eine Gelenkeinrichtung 4 miteinander verbunden.

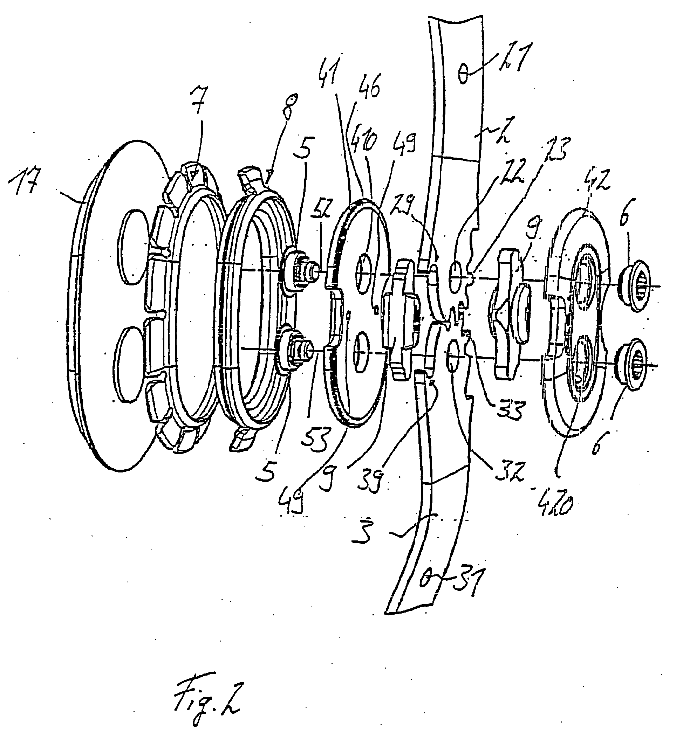

Die Gelenkeinrichtung 4 ist, wie in der Figur 2 besser zu erkennen ist, aus zwei Lagerscheiben 41, 42 aufgebaut, die beidseitig an den Schienen 2, 3 anliegen. Die Lagerscheiben 41, 42 sind über Schrauben 5 und Muttern 6 miteinander gekoppelt, wobei die Schrauben 5 einen Lagerbolzen ausbilden, der durch Bohrungen 23, 33 in den Schienen 2, 3 hindurchgeht. Die Lagerscheiben 41, 42 weisen ebenfalls Durchgangsbohrungen 410, 420 auf, so dass zwei Schwenkachsen 52, 53 für die Schienen 2, 3 ausgebildet werden. An den Lagerscheiben 41, 42 sind umfänglich angeordnete Hinterschnitte 46 ausgebildet, die zur formschlüssigen Aufnahme von Kopplungselementen in Gestalt von Pelottenträgern 7, Adapterringen 8 oder Pelotten 17 dienen.

Wie in der Figur 2 zu erkennen ist, weisen die Schienen 2, 3 Verzahnungen 23, 33 auf, die miteinander kämmen, um eine synchronisierte Verschwenkbewegung zwischen dem Oberteil 2 und dem Unterteil 3 zu realisieren. Dies ist insbesondere bei Kniegelenkorthesen vorteilhaft, bei denen die Schienen 2, 3 sowohl medial als auch lateral neben dem Knie angeordnet sind.

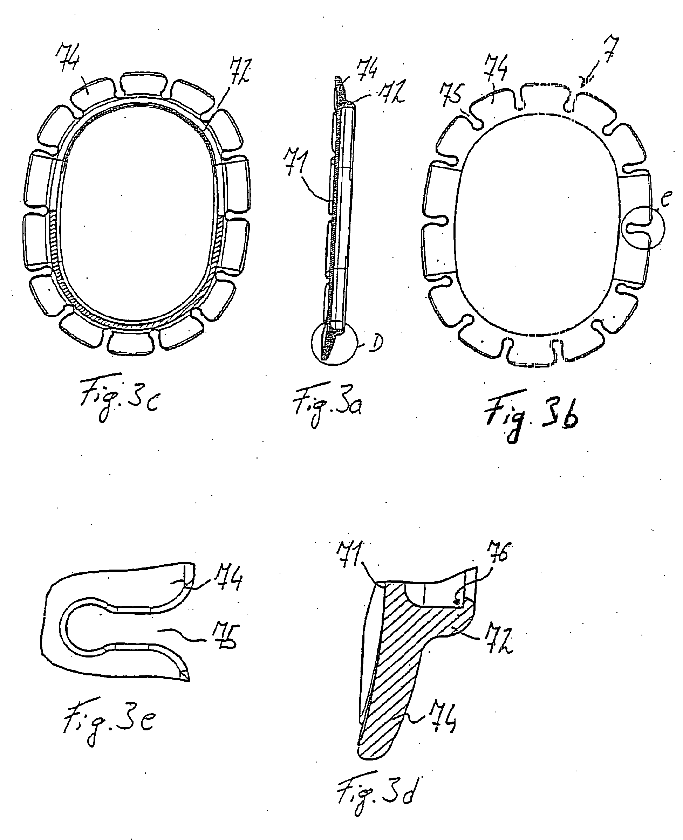

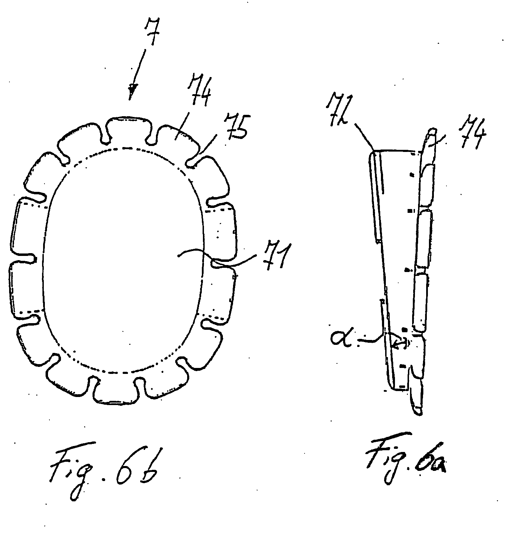

Um den Zwischenraum zwischen dem Gelenk, beispielsweise dem Kniegelenk, und der Gelenkeinrichtung 4 in medialer und lateraler Richtung überbrücken zu können, sind Pelotten 17 vorgesehen, deren Träger in den Zeichnungen 3a bis 3e gezeigt sind. Die Figur 3a zeigt einen Pelottenträger 7 in Querschnittsansicht, der als Kopplungselement zwischen der Pelotte 17 und der Gelenkeinrichtung 4 eingeclipst ist, mit einer dem Gelenk zugewandten Anlageseite 71 und einem ringförmig ausgebildetem Formschlusselement 72, das in der Kontur im Wesentlichen der Kontur der Gelenkeinrichtung 4 entspricht, vorliegend ein Oval ausbildet. Dieses Oval ist in der Rückansicht der Figur 3c zu erkennen. Die Kontaktfläche 71, wie sie in der Draufsicht in der Figur 3b dargestellt ist, ist von einem Rand 74 umgeben, der Einschnitte 75 aufweist, wie in der Figur 3e zu erkennen ist, um eine elastische Anlage an das Kniegelenk oder an eine Polstereinrichtung zu ermöglichen. In der vergrößerten Darstellung gemäß Figur 3d ist in einer Schnittdarstellung die Formschlusseinrichtung 72, die umlaufend ausgebildet ist, zu erkennen. In der Formschlusseinrichtung 72 ist ein Hinterschnitt 76 ausgebildet, der in die korrespondierende Formschlusseinrichtung 46 an der Gelenkeinrichtung 4 eingreift und somit eine Clipsverbindung des Pelottenträgers 7 an der Gelenkeinrichtung 4 ermöglicht. Um den Pelottenträger 7 und analog eine Pelotte 17von der Gelenkeinrichtung 4 zu lösen, muss lediglich die formschlüssige Verriegelung durch Ziehen oder Aufbiegen gelöst und der Pelottenträger 7 oder die Pelotte 17 abgenommen werden. Neben dem in der Figur 3a zu erkennenden, im Wesentlichen geraden Querschnitt sind keilförmige Querschnitte der Pelotte 17 oder des Pelottenträgers 7 vorgesehen, wie sie in der Figur 6a und 6b gezeigt ist, um eine Schrägstellung der Anlagefläche 71 relativ zu den Schwenkachsen oder der Oberfläche der Gelenkeinrichtung 4 realisieren zu können. In der Figur 6a ist der Neigungswinkel α zwischen der Anlagefläche 71 und der Formschlusseinrichutng 72 bzw. der Rückseite des Pelottenträgers 7 zu erkennen.

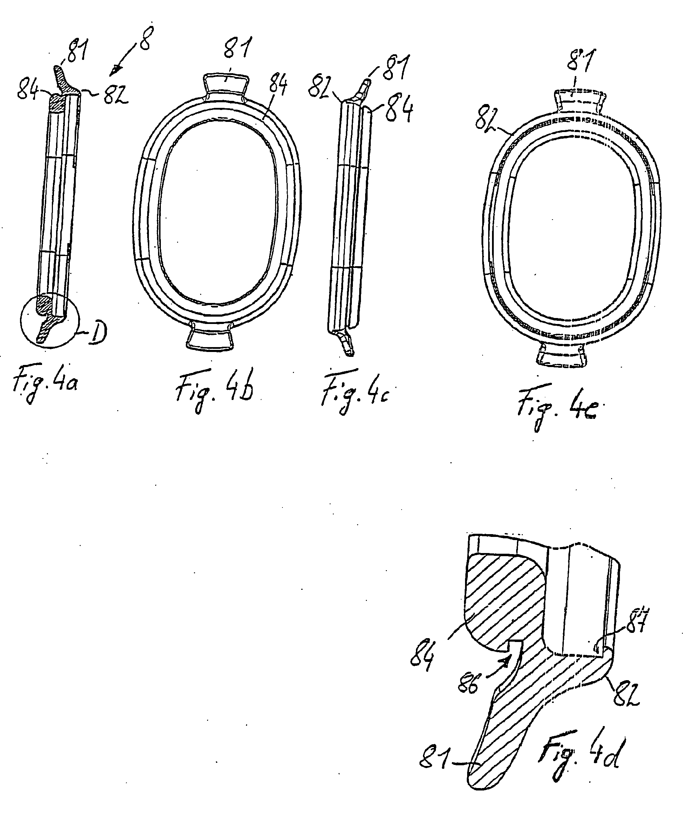

Um eine laterale oder mediale Anpassbarkeit der Pelotte 17 leicht zu erreichen, können Adapter 8, wie sie in den Figuren 4a bis 4e dargestellt sind, zwischen die Pelotte 17 oder die Pelottenträger 7 und die Gelenkeinrichtung 4 eingesetzt werden. Der Adapter 8 weist auf einer Seite eine Formschlusseinrichtung 82 in Gestalt eines hinterschnittenen Ovals auf, die der Formschlusseinrichtung 72 des Pelottenträgers 7 oder einer Pelotte 17 entspricht. Dadurch ist es möglich, den Adapter 8 auf die Gelenkeinrichtung 4 aufzuclipsen. Auf der anderen Seite ist eine Außenkontur 84 ausgebildet, die der Formkontur der Gelenkeinrichtung 4 mit den entsprechenden Formschlusseinrichtungen entspricht. Diese Kontur 84 ist in der vergrößerten Darstellung der Figur 4d besonders gut zu erkennen. Auch hier ist ein Hinterschnitt 86 ausgebildet, der korrespondierend zu dem Hinterschnitt in der Gelenkeinrichtung 4 ausgebildet ist. Der Hinterschnitt 76 der Pelotte oder des Pelottenträgers 7 kann in den Hinterschnitt 86 an dem Adapter 8 formschlüssig eingreifen.

An den distalen und proximalen Enden des Adapters 8 sind Laschen 81 angeordnet, die ein leichtes Lösen des Adapters 8 von der Gelenkeinrichtung 4 oder einem weiteren Adapter 8 durch Anheben erleichtern. Mehrere Adapter 8 können übereinander angeordnet werden, um größere Distanzen zu überbrücken. Neben einem geraden Querschnitt, wie er in der Figur 4a zu erkennen ist, können auch keilförmige Querschnitte analog zu der Ausbildung des Pelottenträgers 7 in der Figur 6 ausgebildet sein.

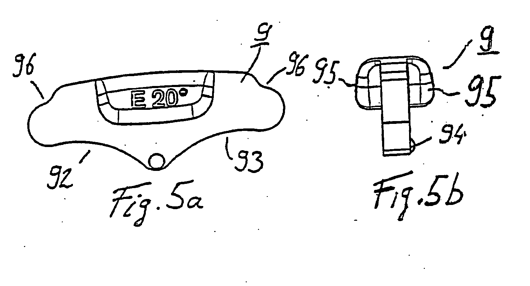

Wie aus der Figur 2 zu erkennen ist, ist der Verzahnung 23, 33 so ausgebildet ist, dass eine Verschwenkbegrenzung in der geraden Stellung, wie sie in der Figur 2 gezeigt ist, auftritt. Dies entspricht einer maximalen Extensions des Kniegelenkes. Um eine Kniegelenksflexion begrenzen zu können, kann in die Gelenkeinrichtung 4 ein Anschlageinsatz 9 eingesetzt werden, der in den Spalt zwischen den Lagerscheiben 41 und 42 eingeschoben wird. Der Anschlageinsatz 9, der in den Figuren 5a und 5b im Detail dargestellt ist, weist Ausnehmungen 92, 93 auf, an denen die Verzahnungen 23, 33 frei entlanggleiten. Zumindest einseitig ist ein Formschlusselement 94 in Gestalt einer Erhöhung ausgebildet, die in eine korrespondierende Ausnehmung 49 der Lagerscheiben 41, 42 eingreift und ein Herausfallen des Anschlageinsatzes 9 im Wesentlichen verhindert. Über Verdickungen 95 kann der Anschlageinsatz 9 leicht gegriffen und wieder aus der Gelenkeinrichtung 4 herausgezogen werden, um veränderte Flexionswinkelbegrenzungen einstellen zu können.

Da bei einer Belastung unter Anschlag der Schienen 2, 3 an dem Anschlageinsatz 9 hohe Kräfte wirken können, die den Anschlageinsatz 9 aus der Gelenkeinrichtung 4 heraustreiben, ist vorgesehen, dass der Pelottenträger 7 oder der Adapter 8 so angeordnet und ausgebildet sind, dass der Anschlageinsatz 9 oder die Verdickung 95 zumindest teilweise umfasst wird, so dass ein Herausdrücken vermieden wird. Ebenfalls ist es vorgesehen, dass an den Schienen 2, 3 zangenartige Formschlusseinrichtungen ausgebildet sind, die in äußere Ausnehmungen 96 in den Anschlageinsätzen 9 eingreifen und somit die Anschlageinsätze zangenartig umgreifen und in der Gelenkeinrichtung 4 fixieren. Ein Austausch der Anschlageinsätze 9 kann dann nicht in maximaler Flexionsstellung folgen.

Grundsätzlich ist die Anordnung von Anschlageinsätzen 9 zur Begrenzung der Extension und Flexion vorgesehen, wie in den Figuren 1 und 2 gezeigt ist, es können jedoch auch nur Flexionsbegrenzungen bei einem festen Extensionswinkel einstellbar sein. Dazu sind die Anschlageinsätze 9 nur auf einer Seite der Schwenkachsen 52, 53 in der Schwenkebene anzuordnen.

## Patentansprüche

1. Gelenkorthese mit einem Oberteil (2) und einem Unterteil (3), die über eine Gelenkeinrichtung (4) um zumindest eine Schwenkachse (52, 53) schwenkbar miteinander verbunden sind, wobei das Oberteil (2) und das Unterteil (3) Einrichtungen zum Festlegen der Gelenkorthese an einem Körperteil, insbesondere an einer Extremität aufweisen, und einer an der Gelenkorthese befestigten Pelotte (17), die in dem Raum zwischen der Gelenkeinrichtung (4) und dem Körperteil angeordnet ist, **dadurch** gekenntzeichnet, dass die Gelenkorthese (1) und die Pelotte (17) oder zumindest ein Kopplungselement (7, 8) Formschlusselemente (46, 76, 86, 87) aufweisen, die eine Clipsverbindung zum Einclipsen der Pelotte (17) auf der Gelenkorthese (1) ausbilden und dass an der Gelenkeinrichtung (4) ein Hinterschnitt (46) zur Aufnahme der Pelotte (17) oder des Kopplungselementes (7, 8) angeordnet oder ausgebildet ist.

2. Gelenkorthese nach Anspruch 1, **dadurch gekennzeichnet, dass** das Oberteil, (2) und das Unterteil (3) Rahmenteile mit Verzahnungen (23, 33) aufweisen, die miteinander kämmen, und die Gelenkeinrichtung (4) für das Oberteil (2) und das Unterteil (3) je eine Schwenkachse (52, 53) ausbildet.

3. Gelenkorthese nach Anspruch 1 oder 2, **dadurch gekennzeichnet, dass** die Pelotte (17) oder das Kopplungselement (7, 8) die Schwenkachsen (52, 53) oder einen Gelenkspalt umgibt, der zwischen Oberteil (2) und Unterteil (3) ausgebildet ist.

4. Gelenkorthese nach einem der voranstehenden Ansprüche, **dadurch gekennzeichnet, dass** das Kopplungselement als ein Adapter (8) und/oder Pelottenträger (7) ausgebildet und zwischen der GelenkeinriChtung (4) und der Pelotte (17) angeordnet ist.

5. Gelenkorthese nach Anspruch 4, **dadurch gekennzeichnet, dass** der Adapter (8), der Pelottenträger (7) oder die Pelotte (17) einen keilförmigen Querschnitt aufweist.

6. Gelenkorthese nach einem der voranstehenden Ansprüche, **dadurch gekennzeichnet, dass** zwei symmetrisch aufgebaute Gelenkeintichtungen (4) einander gegenüberliegend ausgebildet sind, die einen symmetrischen Aufbau aufweisen.

7. Gelenkorthese nach einem der voranstehenden Ansprüche, **dadurch gekennzeichnet, dass** in der Gelenkeinrichtung (4) Aufnahmeelemente (49) für Anschlageinsätze (9) ausgebildet sind.

8. Gelenkorthese nach Anspruch 7, **dadurch gekennzeichnet, dass** der Anschlageinsatz (9) an der Gelenkeinrichtung (4) festgelegt, insbesondere eingeclipst ist.

9. Gelenkorthese nach Anspruch 7 oder 8, **dadurch gekennzeichnet, dass** die Pelotte (17) oder der Pelottenträger (7) oder der Adapter (8) den Anschlageinsatz (9) sichern.

10. Gelenkorthese nach einem der Ansprüche 7 bis 9, **dadurch gekennzeichnet, dass** das Oberteil (2) und/oder das Unterteil (3) einen Hinterschnitt (29, 39) aufweisen, der bei Kontakt mit dem Anschlageinsatz (9) diesen blockiert.

11. Gelenkorthese nach Anspruch 10, **dadurch gekennzeichnet, dass** der Hinterschnitt (29, 39) zangenartig ausgebildet ist.

## Claims

1. Joint orthotic having an upper part (2) and a lower part (3), which are pivotably interconnected about at least one pivot axis (52, 53) via a joint device (4), wherein the upper part (2) and the lower part (3) comprise devices for fixing the joint orthotic to a part of a body, particularly an extremity, and a pad (17) fastened to the joint orthotic which is arranged in the space between the joint device (4) and the part of the body, **characterized in that** the joint orthotic (1) and the pad (17) or at least one coupling element (7, 8) comprise positive connection elements (46, 76, 86, 87) which form a clip-on connection for clipping the pad (17) onto the joint orthotic (1) and **in that** on the joint device (4) an undercut (46) for receiving the pad (17) or the coupling element (7, 8) is arranged or formed.

2. Joint orthotic according to Claim 1, **characterized in that** the upper part (2) and the lower part (3) comprise frame parts with toothings (23, 33), which mesh with each other, and the joint device (4) for the upper part (2) and the lower part (3) forms a pivot axis (52, 53) each.

3. Joint orthotic according to Claim 1 or 2, **characterized in that** the pad (17) or the coupling element (7, 8) surrounds the pivot axes (52, 53) or a joint gap which is formed between upper part (2) and lower part (3).

4. Joint orthotic according to any one of the preceding claims, **characterized in that** the coupling element is designed as an adapter (8) and/or pad carrier (7) and arranged between the joint device (4) and the pad (17).

5. Joint orthotic according to Claim 4, **characterized in that** the adapter (8), the pad carrier (7) or the pad (17) has a wedge-shaped cross section.

6. Joint orthotic according to any one of the preceding claims, **characterized in that** two symmetrically constructed joint devices (4) located opposite each other are formed, which have a symmetrical construction.

7. Joint orthotic according to any one of the preceding claims, **characterized in that** in the joint device (4) receiving elements (49) for stop inserts (9) are formed.

8. Joint orthotic according to Claim 7, **characterized in that** the stop insert (9) is fixed to the joint device (4), particularly clipped in.

9. Joint orthotic according to Claim 7 or 8, **characterized in that** the pad (17) or the pad carrier (7) or the adapter (8) secures the stop insert (9).

10. Joint orthotic according to any one of the Claims 7 to 9, **characterized in that** the upper part (2) and/or the lower part (3) comprise an undercut (29, 39), which upon contact with the stop insert (9) blocks the latter.

11. Joint orthotic according to Claim 10, **characterized in that** the undercut (29, 39) is formed tong-like.

## Revendications

1. Orthèse d'articulation comprenant une partie supérieure (2) et une partie inférieure (2) reliées ensemble pivotantes autour d'au moins une axe de pivotement (52, 53) par l'intermédiaire d'un dispositif d'articulation (4), la partie supérieure (2) et la partie inférieure (3) présentant des dispositifs pour fixer l'orthèse d'articulation sur une partie du corps, en particuller sur une extrémité, et comprenant un tampon (17) fixé à l'orthèse d'articulation et agencé dans l'espace entre le dispositif d'articulation (4) et la partie du corps, **caractérisée en ce que** l'orthèse d'articulation (1) et le tampon (17) ou au moins un élément de couplage (7, 8) présentent des éléments de liaison par formes conjuguées (46, 76, 86, 87) qui forment une liaison par encliquetage pour l'encliquetage du tampon (17) sur l'orthèse d'articulation (1) et **en ce qu'**une contre-dépouille (46) est agencée ou formée sur le dispositif d'articulation (4) pour recevoir le tampon (17) ou l'élément de couplage (7, 8).

2. Orthèse d'articulation selon la revendication 1, **caractérisée en ce que** la partie supérieure (2) et la partie inférieure (3) présentent des parties châssis avec des dentures (23, 33) qui engrènent l'une avec l'autre, et le dispositif d'articulation (4) forme un axe de pivotement (52, 53) respectif pour la partie supérieure (2) et la partie inférieure (3).

3. Orthèse d'articulation selon la revendication 1 ou 2, **caractérisée en ce que** le tampon (17) ou l'élément de couplage (7, 8) entoure les axes de pivotement (52, 53) ou une fente d'articulation qui est formée entre la partie supérieure (2) et la partie inférieure (3).

4. Orthèse d'articulation selon l'une quelconque des revendications précédentes, **caractérisée en ce que** l'élément de couplage est formé par un adaptateur (8) et/ou support de tampon (7) et agencé entre le dispositif d'articulation (4) et le tampon (17).

5. Orthèse d'articulation selon la revendication 4, **caractérisée en ce que** l'adaptateur (8), le support de tampon (7) ou le tampon (17) présente une section en coin.

6. Orthèse d'articulation selon l'une quelconque des revendications précédentes, **caractérisée en ce que** deux dispositifs d'articulation (4) construits symétriquement sont formés l'un en face de l'autre, qui présentent une construction symétrique.

7. Orthèse d'articulation selon l'une quelconque des revendications précédentes, **caractérisée en ce que** des éléments de logement (49) pour des inserts de butée (9) sont formés dans le dispositif d'articulation (4).

8. Orthèse d'articulation selon la revendication 7, **caractérisée en ce que** l'insert de butée (9) est fixé au dispositif d'articulation (4), en particulier encliqueté.

9. Orthèse d'articulation selon la revendication 7 ou 8, **caractérisée en ce que** le tampon (17) ou le support de tampon (7) ou l'adaptateur (8) maintient en place l'insert de butée (9).

10. Orthèse d'articulation selon l'une quelconque des revendications 7 à 9, **caractérisée en ce que** la partie supérieure (2) et/ou la partie inférieure (3) présentent une contre-dépouille (29, 39) qui bloque l'insert de butée (9) par contact avec celui-ci.

11. Orthèse d'articulation selon la revendication 10, **caractérisée en ce que** la contre-dépouille (29, 39) est réalisée en forme de pince.
